# EUROPÄISCHE PATENTSCHRIFT

(11) **EP 2 163 622 B1**
(45) Veröffentlichungstag und Bekanntmachung des Hinweises auf die Patenterteilung: **07.05.2014**
(21) Anmeldenummer: 09011295.4
(22) Anmeldetag: 03.09.2009
(51) Int. Cl.: C12N 15/10

(54) **Verfahren zur Gewinnung von kurzer RNA sowie Kit hierfür**
Method for obtaining short RNA and kit for same
Procédé de production d'ARN courts et kit correspondant

(30) Priorität: 04.09.2008 DE 102008045705
(43) Veröffentlichungstag der Anmeldung: 17.03.2010
(73) Patentinhaber: Macherey, Nagel GmbH & Co. Handelsgesellschaft, 52355 Düren (DE)
(72) Erfinder: Radmacher, Edmund, Dr., 52349 Düren (DE); Möller, Klaus, Dr., 52249 Eschweiler (DE); Meusel, Markus, Dr., 52146 Würselen (DE); Krieger, Robert, Dr., 52349 Düren (DE)
(74) Vertreter: Paul, Dieter-Alfred

(56) Entgegenhaltungen:
- EP-A- 1 529 841
- WO-A-2009/070558
- QIAGEN: "AllPrep® DNA/RNA/Protein Mini Handbook"[Online] Dezember 2007 (2007-12), XP002551264 Gefunden im Internet: URL:http://www1.qiagen.com/literature/rend er.aspx?id=2067> [gefunden am 2009-10-16]
- QIAGEN: "Purification of miRNA from animal cells using the RNeasy® Plus Mini Kit and RNeasy MinElute® Cleanup Kit"[Online] Juni 2006 (2006-06), XP002551265 Gefunden im Internet: URL:http://www1.qiagen.com/literature/rend er.aspx?id=577> [gefunden am 2009-10-20]
- NORGEN BIOTEK CORPORATION: "Non-Organic-Based Isolation of Mammalian microRNA using Norgen?s microRNA Purification Kit" APPLICATION NOTE RNA SAMPLE PREPARATION, [Online] Nr. 13, 2007, XP002551266 Gefunden im Internet: URL:http://www.norgenbiotek.com/tech%20dow nloads/Application%20Notes/Non-Organic-Bas ed%20Isolation%20of%20Mammalian%20microRNA .pdf> [gefunden am 2009-10-15]
- NORGEN BIOTEK CORPORATION: "microRNA Purification Kit"[Online] 29. Juli 2008 (2008-07-29), XP002551267 Gefunden im Internet: URL:http://www.norgenbiotek.com/tech%20dow nloads/Product%20Protocols/PI21300.pdf> [gefunden am 2009-10-16]
- SIGMA-ALDRICH: "More rapid, more pure, less hazard, less hands-on. mirPremier(TM) microRNA Isolation Kit"[Online] 2007, XP002551268 Gefunden im Internet: URL:http://archive.epo.org/epo/pubs/oj009/ 05_09/05_3429.pdf> [gefunden am 2009-10-15]
- INVITROGEN: "PureLink miRNA Isolation Kit"[Online] 18. Juli 2005 (2005-07-18), XP002551269 Gefunden im Internet: URL:http://tools.invitrogen.com/content/sf s/manuals/purelink_microRNA_man.pdf> [gefunden am 2009-10-15]
- QIAGEN: "QIAGEN Supplementary Protocol Sample & Assay Technologies Purification of miRNA from cells and tissues using the AllPrep® DNA/RNA/Protein Mini Kit and RNeasy® MinElute® Cleanup Kit"[Online] November 2008 (2008-11), XP002551274 Gefunden im Internet: URL:http://www1.qiagen.com/literature/rend er.aspx?id=104289> [gefunden am 2009-10-16]
- MACHEREY-NAGEL: "Total DNA, RNA, and Protein Isolation User Manual NucleoSpin® TriPrep"[Online] Januar 2009 (2009-01), XP002551275 Gefunden im Internet: URL:http://www.macherey-nagel.com/Portals/ 8/attachments/Redakteure_Bio/Protocols/RNA %20and%20mRNA/UM_TotalDNARNAProtein.pdf> [gefunden am 2009-10-15]

## Beschreibung

Die vorliegende Erfindung betrifft ein mehrschrittiges Verfahren zur Gewinnung von zumindest kurzer RNA mit bis zu 200 Nukleotiden sowie einen Kit hierfür.

In den letzten Jahren hat sich herausgestellt, dass es sich bei kurzen RNAs in Zellen nicht um unwichtige Abbauprodukte langer RNA, z. B. von rRNA oder mRNA handelt, sondern dass diese gezielt synthetisiert werden. Sie spielen eine essenzielle Rolle in der Genregulation, RNA Synthese, RNA Modifikation und dem RNA Splicing in nahezu allen lebenden Organismen, und es werden kontinuierlich neue Funktionen und damit Anwendungsfelder entdeckt. Entsprechend ihrer Funktion, Struktur, Lokalisation in der Zelle oder Reaktionspartner werden diese kleinen RNAs in Gruppen wie z.B. miRNA, siRNA, shRNA, snoRNA, scnRNA, piRNA, tasiRNA, rasiRNA etc. unterteilt, deren Zahl ebenfalls ständig anwächst.

Die kurzen RNAs liegen meist einzelsträngig vor und weisen typischerweise Längen im Bereich von 17-35 Nukleotiden auf. Es gibt aber auch kurze Doppelstrang-RNAs wie z.B. siRNA oder shRNA, die jedoch zur Entfaltung ihrer Wirkung ebenfalls in Einzelstränge überführt werden.

Während für die meisten kurzen RNAs die Biosynthese und Funktionsweise noch nicht vollständig erschlossen ist, konnten für einige Spezies bereits konkrete wirtschaftlich und wissenschaftlich interessante Anwendungen entwickelt werden. Von besonderem Interesse sind hierbei miRNA und siRNA.

MiRNAs (micro RNAs) sind kernkodiert, werden in der Zelle synthetisiert und führen über verschiedene Mechanismen in der Regel zur gezielten Repression der Genexpression. Die betroffenen Gene sind dabei vor allem an der Zellentwicklung, -teilung und -differenzierung beteiligt. Es konnte gezeigt werden, dass sich das miRNA Expressionsprofil gesunder Zellen deutlich von dem unterscheidet, das man in Zellen mit gestörtem Wachstum bei z. B. bei Krebs findet. Mit einem chipbasierten "miRNA Profiling", d.h. der Quantifizierung eines Sets von miRNAs, ist mittlerweile eine viel genauere Identifikation einer (Krebs-)Erkrankung möglich als es bisherige Methoden erlaubten.

Die Bildung doppelsträngiger siRNA (short interfering RNA) wird in fast allen pflanzlichen und tierischen Zellen zur Abwehr von Pathogenen eingesetzt. Nach dem Eindringen eines Pathogens in die Zelle kommt es meist zu einer intermediären Bildung doppelstängiger mRNA, die durch zelleigene Dicer-Enzymkomplexe in die kleine siRNA zerschnitten wird. Die siRNA selbst dient dann als sequenzspezifische Sonde für die zellfremde mRNA, die dadurch von speziellen Nukleasekomplexen (RISC) identifiziert, geschnitten und so unschädlich gemacht wird. Diese Repression auf mRNA Ebene kann nun eingesetzt werden, um beliebige, also auch zelleigene Gene und damit v, a. die entsprechenden Proteine in ihrer Expression zu drosseln. Dazu werden beispielsweise in vitro synthetisierte siRNA in die Zellen eingebracht, oder es wird über Vektorsysteme für eine intrazelluläre siRNA-Synthese gesorgt. Die kurzen RNAs in den Zellen bewirken, dass die Zielgene, die durch die Sequenz der kurzen RNAs vorgegeben sind, durch die oben beschriebenen Mechanismen reprimiert werden. Damit eröffnet sich eine sehr elegante und einfache Möglichkeit, einzelne Gene gezielt zu inaktivieren, was großen Wert bei der Untersuchung von Genregulation und Proteinfunktion hat. Damit ist diese Methode den herkömmlichen Verfahren, wie beispielsweise der Herstellung von Knock-out Mutanten, hinsichtlich Kosten, Zeitaufwand und genereller Machbarkeit deutlich überlegen.

Für die genannten Anwendungen wie Transfektion mit siRNA oder "miRNA Profiling", aber auch für die weitere Entschlüsselung von Struktur und Funktionsmechanismen ist es erforderlich, die wirkenden RNA Spezies möglichst quantitativ aus den Probenmaterialien aufzureinigen. Zusätzlich muss die kurze RNA für die immer empfindlicher werdende Nachfolgeanalytik weitgehend von einem sehr großen Hintergrund an langer RNA, DNA und Protein befreit werden. Dies stellt höchste Anforderungen an die Extraktionsmethode, da die kurzen RNAs oftmals nur in Spuren vorliegen.

Nach dem heutigen Stand der Technik basiert fast jede Reinigung von kleiner RNA auf dem Prinzip, aus dem Lysat einer biologischen Probe zuerst die großen Makromoleküle wie DNA, lange RNA und teilweise auch Protein zu entfernen. Die kleine RNA wird im Anschluss durch Alkohol gefällt und dann meist über die Bindung an einen festen Träger isoliert. Der kritische Punkt bei diesem Verfahren ist die möglichst vollständige Entfernung des Proteins vor der Fällung kleiner RNA, da Protein sonst copräzipitiert und eine weitere Reinigung der kleinen RNA sehr erschwert.

Die WO 2005/012523 A1 beschreibt ein Verfahren zur Gewinnung kurzer RNA mit 100 Nukleotiden oder weniger, bei dem die Entfernung von DNA und Protein über eine Flüssig-Flüssig-Extraktion mit Phenol/Chloroform durchgeführt wird. Sämtliche RNA befindet sich nach der Extraktion in der wässrigen Phase, die anschließend von der Mehrheit der Proteine enthaltenden, organischen Phase und der die DNA enthaltenden Interphase getrennt werden muss. Danach wird durch Zugabe von Alkohol zur wässrigen Phase eine Bedingung geschaffen, unter der die RNA an einen festen Träger bindet.

Bei diesem Verfahren ist von Nachteil, dass die Verwendung von Phenol ein beträchtliches gesundheitliches Risiko für den Anwender in sich birgt, da Phenol giftig und ätzend ist. Zudem kann es sich als schwierig erweisen, nach der Flüssig-Flüssig-Extraktion die wässrige Phase quantitativ abzunehmen, ohne hierbei Spuren der Interphase mit DNA oder der phenolischen Phase mit Protein zu verschleppen. Wird die wässrige Phase aus diesem Grund nicht vollständig entfernt, müssen beträchtliche Ausbeuteverluste an RNA in Kauf genommen werden. Ein weiterer Nachteil dieses Verfahrens kann darin bestehen, dass die Flüssig-Flüssig-Extraktion und die damit verbundenen Phasentrennung nicht ohne weiteres automatisiert werden kann. Vor allem in Hinblick auf mögliche zukünftige Routinediagnostik mittels "miRNA-Profiling, stellt die umständliche und langwierige Prozedur und die Limitierung des Probendurchsatzes durch manuelle Bearbeitung einen gravierenden Nachteil dar.

Andere Verfahren verzichten daher auf die Extraktion mit organischen Lösungsmitteln. So wird in der WO 2005/012487 A2 ein Verfahren vorgeschlagen, bei dem lange RNA und DNA mittels Säulenchromatographie in Gegenwart von kleinen Mengen an Alkohol abgetrennt wird. Die kleine RNA verbleibt in Lösung und wird nach Erhöhen des Alkoholgehaltes an einen zweiten Träger gebunden.

Nachteilig bei diesem Verfahren ist, dass ebenfalls in der Lösung befindliche Proteine durch das Verfahren nicht vollständig abgetrennt werden. Bei einer Erhöhung des Alkoholgehaltes zur Abtrennung der kleinen RNA können die Proteine, wie oben beschrieben, co-präzipitieren und mit der kleinen RNA an den Träger binden. Ein vollständiges Auswaschen des Proteins ist dann kaum mehr möglich. Ein weiterer Nachteil ergibt sich daraus, dass sich unter den zellulären Proteinen häufig auch RNasen befinden, die ohne quantitative Abreinigung die isolierten RNAs verdauen und damit die Ausbeute schmälern bzw. durch Abbauprodukte verunreinigen, im schlimmsten Fall sogar unbrauchbar machen.

Ein weiteres Verfahren zur Reinigung kleiner RNA Spezies wird in US 2007/0202511 A1 beschrieben. Durch gleichzeitiges oder sequenzielles Versetzen einer RNA enthaltenden Lösung mit einem chaotropen Reagenz und Metallsalzen der I. und II. Hauptgruppe des Periodensystems werden lange RNAs und genomische DNA präzipitiert, während kurze RNA Moleküle in Lösung verbleiben. Anschließend wird der die kurze RNA enthaltende Überstand vom Präzipitat getrennt, und die kurzen RNAs werden weiter aufgereinigt. Hierzu kommen Zentrifugationsverfahren oder verschiedene chromato-graphische Verfahren zum Einsatz.

Nachteilig bei diesem Verfahren ist, dass die vollständige Proteinentfernung auch in diesem Fall nicht gewährleistet ist. Das Verfahren erfordert eine anschließende chromatographische Reinigung der kleinen RNA, für die wieder eine alkoholische Fällung vorgeschlagen wird, deren Problem in Gegenwart von Protein bereits erläutert wurde. Eine gesonderte Abtrennung der Proteine ist nicht vorgesehen.

Ein Verfahren, das zu einer Entfernung des Proteins führt, jedoch ohne Flüssig-Flüssig-Extraktion mit Phenol auskommt, findet sich in dem kommerziellen Produkt "All-Prep DNA/RNA/Protein" der Firma Qiagen, Hilden (DE) zur Reinigung von DNA, langer RNA und Protein. Dabei wird DNA in Gegenwart von Chaotropsalz an eine erste Silicamembran gebunden. Nach Zugabe von Alkohol kann nun auch die lange RNA an eine zweite Silicamembran gebunden werden. Das verbleibende Lysat wird zur Gewinnung des Proteins mit einer Zink enthaltenden Lösung versetzt. Das Protein wird dabei gefällt und kann durch Zentrifugation isoliert werden.

Dieses Verfahren ist nicht zur Reinigung kurzer RNA gedacht oder geeignet, da die in dem vorgeschlagenen Kit verwendete Bindechemie im Zusammenspiel mit Silicamembranen zu einem Cut-off von ca. 200 Nukleotiden führt, so dass kurze RNA im Laufe der Aufreinigung verloren geht.

Aus einem weiteren kommerziellen Produkt "Rneasy^{®} Plus Mini Kit and Rneasy MinElute^{®} Cleanup Kit" der Firma Qiagen, Hilden (DE) ist ein Verfahren zur Auftrennung langer und kurzer RNA bekannt. Dieses Verfahren ist allerdings nicht zur quantitativen Gewinnung kurzer RNA hoher Reinheit bei Anwesenheit von gelösten Proteinen geeignet, da für letztere keine Abtrennung vorgesehen ist.

Der vorliegenden Erfindung liegt die Aufgabe zugrunde, ein Verfahren zu schaffen, das eine möglichst quantitative Gewinnung kurzer RNA aus einer Probe ermöglicht, dabei gleichzeitig weitestgehend ohne giftige Chemikalien auskommt. Ferner sollte das Verfahren geeignet sein, in einem automatisierten Verfahren durchgeführt werden zu können, d.h. ohne die schwer zu automatisierende Flüssig-Flüssig-Extraktion z.B. mit Phenol/Chloroform. Dabei sollte die gewonnene kurze RNA auch einen hohen Reinheitsgrad aufweisen. Ferner sollte das Verfahren die Möglichkeit schaffen, die weiteren Bestandteile einer solchen Probe, insbesondere lange Nukleinsäuren und Proteine gesondert zu gewinnen, wobei auch diese möglichst quantitativ und in hoher Reinheit erhalten werden sollten. Eine weitere Aufgabe besteht darin, einen für die Durchführung des Verfahrens geeigneten Kit bereitzustellen.

Der erste Teil der Aufgabe wird erfindungsgemäß durch ein Verfahren mit folgenden Schritten gelöst:
a) Bereitstellen einer biologischen Lösung, enthaltend zumindest kurze RNA sowie Proteine und/oder lange Nukleinsäuren (lange RNA und DNA);
b) Entfernung der Proteine und der langen Nukleinsäuren aus der Lösung, wobei zumindest die Proteine gefällt werden und die Lösung zur Fällung der Proteine mit zweiwertigen Metallionen versetzt wird, wobei die Konzentration der Metallionen auf 0,2 bis 0,6 M eingestellt wird;
c) Adsorbieren der kurzen RNA an einem festen (ersten) Träger nach dem Fällen der Proteine;
d) Gewinnung der kurzen RNA durch Desorption von dem Träger.

Überaschenderweise wurde gefunden, dass bei einem Verfahren, bei dem zumindest zunächst die Proteine aus der RNA enthaltenden Lösung gefällt werden und anschließend die kurze RNA an einem Träger adsorbiert wird, von dem sie in einem weiteren Schritt wieder desorbiert werden kann, die enthaltene kurze RNA mit hoher Ausbeute bei gleichzeitig hoher Reinheit erhalten wird.

Wären zum Zeitpunkt der Adsorption der kurzen RNA am ersten Träger noch größere Mengen an Proteinen vorhanden, so würden diese unter den entsprechenden Bedingungen ebenfalls ausfallen und an der Bindematrix binden. Im schlimmsten Fall käme es zur Verstopfung der Bindematrix und einer erheblichen Verunreinigung der kurzen RNA mit Protein. Nach dem erfindungsgemäßen Verfahren ist eine solche Proteinverschleppung und/oder -ausfällung durch die vorherige Fällung des Proteins nahezu ausgeschlossen, und die nachfolgenden Waschschritte können auf ein Minimum reduziert werden.

Ein weiterer Vorteil des erfindungsgemäßen Verfahrens besteht in der Möglichkeit, Proteine in einer Form zu gewinnen, dass sie direkt für weitere Zwecke verwendbar sind. Dies ist bei allen übrigen Verfahren zur,Gewinnung kurzer RNAs nicht der Fall.

Unter kurzer RNA werden im Rahmen der vorliegenden Erfindung RNA Moleküle verstanden, welche typischerweise bis zu 200 Nukleotide aufweisen, insbesondere bis zu 150 Nukleotide oder bis zu 100 Nukleotide oder gar nur bis zu 75 Nukleotide oder nur 40 Nukleotide. Die kurze RNA soll durch das erfindungsgemäße Verfahren insbesondere von langer RNA, DNA und Proteinen abgetrennt werden.

Kurze RNA unterscheidet sich im Rahmen dieser Erfindung von langen RNAs wie etwa ribosomale rRNA (rRNA) oder messenger-RNA (mRNA). Kleine RNAs im Sinne dieser Erfindung sind beispielsweise 5.8S rRNA, 5S rRNA, transfer RNAs (tRNA), small nuclear RNA (snRNA), small nucleolar RNA (snoRNA), micro RNA (miRNA), small interfering RNA (siRNA), trans-acting siRNA (tasiRNA), repeat-associated siRNA (rasiRNA), small temporary RNA (stRNA), tiny noncoding RNA (tncRNA), small scan RNA (scRNA) und small modulatory RNA (smRNA).

Als Quelle für die zu extrahierenden kurzen RNAs können natürliche biologische Materialien wie Zellen, Gewebe, Organe, Organismen, Körperflüssigkeiten etc. dienen, ebenso wie in vitro Reaktionsgemische, bei denen RNA Moleküle produziert, geschnitten oder modifiziert werden, wie beispielsweise ein in vitro Verdau langer dsRNA durch Dicer. Grundsätzlich ist das erfindungsgemäße Verfahren prinzipiell auf alle kleine RNA enthaltende Proben anwendbar.

Zum Bereitstellen der biologischen Lösung werden die Proben zunächst einer Lyse unterworfen. Hierzu werden üblicherweise Lysepuffer mit chaotropen Reagenzien eingesetzt. Chaotrope Reagenzien sind dem Fachmann bekannt. Sie zerstören Membranen, denaturieren Proteine, führen zur Zelllyse und setzen Nukleinsäuren frei. Gemäß Ihrer Tendenz, hydrophobe Wechselwirkungen zu schwächen, werden Ionen in der s. g. Hofmeisterreihe angeordnet. Ionen mit ausgeprägtem chaotropen Charakter sind z. B. Guanidinium, Barium und Calcium. Häufig bei der Nukleinsäurereinigung eingesetzte Chaotropsalze sind u. a. Guanidiniumhydrochlorid, Guanidiniumthiocyanat oder Natriumperchlorat. Die beschriebenen Salze führen bei der Lyse zu einem Auflösen von Zellen oder Zellverbänden unter gleichzeitiger Inaktivierung von RNasen. Die Lyse kann zudem nach dem Stand der Technik mechanisch unterstützt werden. Hierzu kann der Fachmann auf eine Vielzahl von Möglichkeiten wie etwa Rotor/Stator-Systeme, Mörser, Kugelmühlen, etc. zurückgreifen. Nach der Lyse schließen sich üblicherweise Verfahren an, bei denen unlösliche (Zell-)Bestandteile durch beispielsweise Filtration oder Zentrifugation entfernt werden.

Die Fällung der Proteine aus der Lösung erfolgt vor der Adsorption der kurzen RNA an dem festen Träger. Dabei können mit der Fällung der Proteine auch die langen Nukleinsäuren entfernt werden. Insbesondere dann, wenn die langen Nukleinsäuren für weitere Zwecke gewonnen werden sollen, empfiehlt es sich jedoch, die langen Nukleinsäuren vorzugsweise vor der Fällung der Proteine an einen festen (zweiten Träger) zu adsorbieren. Auf diese Weise ist eine weitestgehend vollständige Abtrennung der langen Nukleinsäuren möglich und besteht darüber hinaus die Möglichkeit, diese getrennt von den Proteinen einer weiteren Verwertung zuzuführen. Hierzu werden die langen Nukleinsäuren von dem festen (zweiten) Träger desorbiert, insbesondere eluiert, ggf. nach zumindest einem Waschvorgang.

Durch das Verfahren wird es nun erstmals möglich, Protein, lange RNA und kurze RNA aus einer einzigen, ungeteilten Probe in hoher Reinheit und vor allem auch quantitativ zu isolieren. Hierdurch eröffnet das Verfahren die Möglichkeit einer zuverlässigen quantitativen Bestimmung sowohl von kurzer RNA, aber auch von langen Nukleinsäuren und Proteinen. Dies ist für die Untersuchung der Genexpression ein entscheidender Fortschritt, da die Menge vieler kleiner RNAs (z. B. miRNA) einen regulatorischen Einfluss auf die Menge vieler mRNAs hat (lange RNA), die wiederum die Menge des jeweils exprimierten Proteins mitbestimmt. Doch nicht nur bei der Untersuchung natürlicher Regulationsprozesse ist die genaue Kenntnis der vorhandenen Mengen der drei Komponenten wichtig. Mittels kleiner RNAs (z. B. siRNA), die von außen in die Zelle eingebracht werden, lassen sich mRNA und Proteingehalt regeln. Für die exakte Untersuchung einer Dosis-Wirkungsbeziehung ist wiederum die Kenntnis der in einer Probe vorhandenen Mengen an kleiner RNA, langer RNA und Protein erforderlich.

Dem Lysat sollte ein insbesondere wassermischbares, organisches Lösungsmittel in einer Menge zugegeben werden, die ausreicht, lange Nukleinsäuren (RNA und DNA) an den Träger zu binden. In bevorzugter Weise wird die Lösungsmittelkonzentration auf 15-40%, besonders bevorzugt auf 20-30%, noch besser auf 25%, jeweils bezogen auf die Lösung insgesamt, eingestellt. Diese Konzentrationen führen zu einer guten Adsorption der langen Nukleinsäuren, belassen aber die kurze RNA in der Lösung. Bevorzugte organische Lösungsmittel sind ein oder mehrere Alkohole, insbesondere Ethanol und/oder 2-Propanol.

Eine weitere Ausgestaltung des erfindungsgemäßen Verfahrens sieht vor, die Lösung zur Adsorption der langen Nukleinsäuren mit einem Salz hoher Konzentration zu versetzen. Hierzu eignet sich besonders ein chaotropes Salz oder eine Mischung mehrerer chaotroper Salze. Dabei werden unter dem Begriff "hoher Konzentration" Salzkonzentrationen von größer/gleich 1 M verstanden. Besonders vorteilhaft ist es, wenn die Konzentration des Salzes in der Lösung auf einen Bereich von 1 bis 10 M eingestellt wird.

Sofern lange RNA isoliert gewonnen werden soll, ist es zweckmäßig, die DNA von den an dem zweiten Träger adsorbierten Nukleinsäuren zu entfernen. Dies kann beispielsweise durch DNase-Verdau erfolgen. Die lange RNA kann dann, gegebenenfalls nach einem waschvorgang mit geeigneten Waschpuffern, von dem zweiten Träger eluiert werden.

Zur Entfernung der Proteine wird die Lösung, die die kurzen, nicht gebundenen RNA Spezies enthält, mit einem Protein-fällenden Reagenz versetzt. Bevorzugt werden die Proteine mit einer zweiwertige Metallionen enthaltenden Lösung gefällt. Hierzu werden Salze der entsprechenden Metalle eingesetzt. Geeignete Metalle in diesem Sinne sind z.B. Fe, Co, Ni, Cu, Zn, Cd, Hg, Pb und Ba, zweckmäßigerweise in einem Konzentrationsbereich (wirksame Endkonzentration) von 0,01 bis 1, 5 M, besser 0,05 bis 1 M, noch besser 0,1 bis 0,8 M, besonders bevorzugt 0,2 bis 0,6 M und optimalerweise 0,30 bis 0,40 M.

Die Lösung wird anschließend durch Zentrifugation oder durch Passieren einer geeigneten Filtervorrichtung von dem präzipitierten Protein befreit. Der Durchfluss ist nun weitgehend frei von Protein. Das abgetrennte Protein steht direkt für weitere Analysen zur Verfügung. So kann es z. B. in bekannter Weise in Laemmli-Puffer gelöst, quantifiziert, einer Gelelektrophorese unterzogen und in Western Blots eingesetzt werden. Überraschenderweise wurde nun festgestellt, dass diese Proteinfällung, die auch geeignet ist, lange RNA oder DNA zu präzipitieren und deshalb bisher nur nach der Entfernung von RNA und DNA eingesetzt wurde, die kleinen RNA Spezies nicht erfasst.

Die Adsorption der kurzen RNA an einem festen (ersten) Träger nach dem Fällen der Proteine wird dadurch gefördert bzw. erreicht, dass zu der Lösung weitere organische, mit Wasser mischbare Lösungsmittel bis zum Erreichen einer hohen Konzentration hinzu gegeben werden, in bevorzugter Weise bis zum Erreichen einer Endkonzentration von 30 bis 80 Vol.%, vorzugsweise 40 bis 70 Vol.%, insbesondere von etwa 50 bis 60 Vol.%, jeweils bezogen auf die Gesamtlösung.

Besonders geeignete Lösungsmittel sind in diesem Fall nicht-alkoholische Lösungsmittel. Vertreter dieser Gruppe sind beispielsweise Aceton, Acetonitril, Dimethylsulfoxid (DMSO), Tetrahydrofuran (THF), Dioxan und Dimetyhlformamid (DMF). Diese können einzeln oder auch als Mischung untereinander eingesetzt werden. Diese Lösungsmittel ermöglichen eine gute Adsorption der kurzen RNA am Träger. Ein weiterer Vorteil der Verwendung nichtalkoholischer, organischer Lösungsmittel zur Fällung der kurzen RNA besteht darin, dass auf diesem Wege eine Reduktion der Kontamination der kurzen RNA durch Proteine erreicht wird.

Zur Gewinnung der kurzen RNA durch Desorption von dem Träger wird der Träger gewaschen und die gebundenen kurzen RNA Moleküle anschließend in hochreiner, konzentrierter Form eluiert.

Als fester Träger werden feste Phasen verstanden, die wasserunlöslich sind und an die Nukleinsäuren in wässriger Phase vorzugsweise mit hoher Ionenstärke binden. Beispiele sind poröse oder nicht-poröse Partikel wie Silica, Glass, Quarz, Zeolithe oder Mischungen daraus. Der Begriff feste Phase kann zudem magnetische oder nicht-magnetische Partikel, Polymere und Materialien bezeichnen, die beispielsweise mit Silica, Glas, Quarz oder Zeolithen beschichtet sind. Der feste Träger kann zudem in Form von Pulver oder Suspensionen vorliegen oder aber in Form einer Membran, einer Filterschicht, einer Fritte, eines Monolithen oder eines anderweitigen festen Körpers ausgebildet sein. Erster und zweiter fester Träger können beim erfindungsgemäßen Verfahren und beim erfindungsgemäßen Kit identisch oder verschieden sein.

Bevorzugt werden als feste Träger Silicamembranen oder Glasfaservliese verwendet. Werden feste Träger in Form von Membranen oder Vliesen verwendet, kann der Fluss durch diese Bindematrices durch Gravitation, Zentrifugieren oder durch Anlegen eines Vakuums erfolgen.

Eine besondere Ausführungsform des erfindungsgemäßen Verfahrens sieht vor, die RNA-Bindematrix in Hohlkörpern mit Ein- und Auslassöffnung ein- oder mehrschichtig zu fixieren. Solche Hohlkörper sind dem Fachmann beispielsweise als Minispin-Zentrifugenkörper bekannt. Alternativ kann die Bindematrix aber auch in Form von magnetischen oder nicht-magnetischen Partikeln vorliegen. Während bei den Minispin-Säulen die Binde-, Wasch-, Separations- und Elutionsschritte durch Zentrifugalkraft vermittelt werden, kann man bei Verwendung magnetischer Beads auf eine Magnetseparation zurückgreifen. Entsprechende Vorrichtungen sind dem Fachmann bekannt. Werden nicht-magnetische Beads verwendet, so kann die Separation durch Sedimentation oder Zentrifugation erfolgen.

Bei partikulären festen Trägern erfolgt eine Inkubation des Lysates mit der Bindematrix und anschließend die Abtrennung des festen Trägers. Dies kann, wie oben beschrieben, beispielsweise durch Sedimentation, Zentrifugation oder Filtration erfolgen. Dies gilt sowohl für erste als auch zweite feste Träger im Rahmen des erfindungsgemäßen Verfahrens.

Ein weiterer Gegenstand der vorliegenden Erfindung ist ein Kit für die Durchführung des erfindungsgemäßen Verfahrens, enthaltend
a) zumindest einen festen Träger zur Adsorption von Nukleinsäuren;
b) ein proteinfällendes Reagenz enthaltend zweiwertige Metallionen;
c) zumindest eine Bindesubstanz zur selektiven Absorption von kurzer RNA an einem festen Träger, wobei die Bindesubstanz ein wassermischbares und nicht-alkoholisches organisches Lösungsmittel ist;
e) eine Anleitung mit Beschreibung der Verfahrensschritte des erfindungsgemäßen Verfahrens.

Mit Hilfe des erfindungsgemäßen Kits wird dem Benutzer neben einer Zusammenstellung aller für die Durchführung des erfindungsgemäßen Verfahrens erforderlichen Chemika-lien und Materialien eine Anleitung an die Hand gegeben, die die Gefahr von Anwendungsfehlern reduziert und auf diese Weise den Erfolg bei der praktischen Durchführung des erfindungsgemäßen Verfahrens sicherstellen kann. Die Anleitung gemäß Merkmal e) kann beispielsweise in schriftlicher Form oder auf einer CD oder DVD gespeichert vorliegen.

Nach einer bevorzugten Ausführungsform ist in dem erfindungsgemäßen Kit zumindest eine Bindesubstanz zur Einstellung der Bindebedingungen für die selektive Adsorption von langen Nukleinsäuren an einem festen Träger vorhanden. Als Bindesubstanz kommen für diesen Zweck die näher beschriebenen organischen Lösungsmittel in Betracht, insbesondere ein wassermischbares, organisches Lösungsmittel, das einen oder mehrere Alkohole, vor allem Ethanol und/oder Propanol, umfasst.

Bei einer weiteren Ausführungsform ist in dem erfindungsgemäßen Kit als zumindest eine Bindesubstanz ein Salz aus einem oder mehreren chaotropen Salzen vorhanden. Das Kit kann ferner eine DNase für einen DNA-Verdau aufweisen.

Nach einer weiteren bevorzugten Ausführungsform weist das Kit als proteinfällendes Reagenz wenigstens eine Metallionen enthaltende Verbindung auf. Dies sind in der Regel Metallsalze. Dabei ist die Metallionen enthaltende Verbindung bevorzugt ausgewählt ist aus Verbindungen bzw.

Salzen, die die Elemente Fe, Ce, Ni, Cu, Zn, Cd, Hg, Pb und/ oder Ba beinhalten.

Eine weitere Zusammenstellung des erfindungsgemäßen Kits zeichnet sich dadurch aus, dass als zumindest eine Bindesubstanz für die Adsorption von kurzer RNA ein organisches Lösungsmittel vorhanden ist. Dabei handelt es sich bevorzugterweise um ein wassermischbares nicht-alkoholisches Lösungsmittel, insbesondere Aceton, Acetonitril, Dimethylsulfoxid (DMSO), Tetrahydrofuran (THF), Dioxan und Dimethyl-formamid (DMF) oder Mischungen von diesen.

Für den erfindungsgemäßen Kit kommen die schon erwähnten festen Träger in Frage, die für die Adsorption der Nukleinsäuren geeignet sind.

Das Kit kann ferner zumindest ein Waschpuffer beinhalten sowie zumindest ein Elutionsreagenz zur Desorption zumindest der kurzen RNA von dem Träger.

### AUSFÜHRUNGSBEISPIELE

Bevorzugte Ausführungsformen der vorliegenden Erfindung werden nun anhand von Ausführungsbeispielen im Zusammenhang mit zugehörigen Figuren im Einzelnen beschrieben. Dabei zeigen:
- Figur 1: ein Elektropherogramm einer Fraktion langer RNA aus Schweineleber, gereinigt nach dem erfindungsgemäßen Verfahren über Silicamem-bransäulen;
- Figur 2: ein Elektropherogramm einer Fraktion kurzer RNA aus Schweineleber, gereinigt nach dem erfindungsgemäßen Verfahren über Silicamem-bransäulen;
- Figur 3: eine tabellarische Zusammenfassung der über UV-VIS (A260, A280) bestimmten RNA Ausbeuten und Reinheiten sowie des Cp-Wertes einer miR-16 qRT-PCR Quantifizierung des 1:10⁵ verdünnten Eluates. Die Fraktionen langer und kurzer RNA aus Schweineleber wurden nach dem erfindungsgemäßen Verfahren über Silicamem-bransäulen gewonnen;
- Figur 4: ein SDS-PAGE der aus Schweineleberlysat nach dem erfindungsgemäßen Verfahren gefällten Proteine. Spur 1; Low Molecular weight Marker, Spur 2, 3; 10 µl Protein (3 µg/µl), Spur 4, 5: 5 µl Protein (3 µg/µl);
- Figur 5: ein Elektropherogramm einer Fraktion langer RNA aus HeLa Zellen, gereinigt nach dem erfindungsgemäßen verfahren über Silica-partikel;

- Figur 6: ein Elektropherogramm einer Fraktion kurzer RNA aus HeLa Zellen, gereinigt nach dem erfindungsgemäßen Verfahren über Silica-partikel;
- Figur 7: eine tabellarische Zusammenfassung der über UV-VIS (A260, A280) bestimmten RNA Ausbeuten und Reinheiten sowie des Cp-Wertes einer miR-16 qRT-PCR Quantifizierung des 1:10⁵ verdünnten Eluates. Die Fraktionen langer und kurzer RNA aus HeLa Zellen wurden nach dem erfindungsgemäßen Verfahren über Silicapartikel gewonnen;
- Figur 8: ein SDS-PAGE der aus HeLa Zellen Lysat nach dem erfindungsgemäßen Verfahren gefällten Proteine. Spur 1: Low Molecular Weight Marker, Spur 2, 3: 10 µl Protein (0,5 µg/µl);
- Figur 9: ein 1% TAE Agarose Gel eines gereinigten in vitro DICER Reaktionsansatzes. Spur 1: 100 bp Marker, Spur 2, 3: 30 µl gereinigte ungeschnittene dsRNA, Spur 4: 30 µl ungereinigter Reaktionsansatz, Spur 5, 6: 30 µl gereinigte siRNA;

- Figur 10: die RNA Ausbeute aus 30 mg Schweineleber ohne und mit vorheriger Fällung des Proteins gemäß dem erfindungsgemäßen Verfahren;
- Figur 11: ein Elektropherogramm der Fraktion kurzer RNA aus Schweineleber, gereinigt nach dem erfindungsgemäßen Verfahren nach Protein-Fällung mit 200 mM Zn;
- Figur 12: ein Elektropherogramm der Fraktion kurzer RNA aus Schweineleber, gereinigt nach dem erfindungsgemäßen Verfahren nach Protein-Fällung mit 400 mM Zn;
- Figur 13: ein Elektropherogramm der Fraktion kurzer RNA aus Schweineleber, gereinigt nach dem erfindungsgemäßen Verfahren nach Protein-Fällung mit 600 mM Zn;
- Figur 14: eine tabellarische Zusammenfassung der über UV-VIS (A260, A280) bestimmten RNA Ausbeuten und Reinheiten sowie des Cp-Wertes einer miR-16 qRT-PCR Quantifizierung des 1:10⁵ verdünnten Eluates. Die Fraktionen langer und kurzer RNA aus Schweineleber wurden nach dem erfindungsgemäßen Verfahren über Silicamembransäulen gewonnen;

- Figur 15: eine Darstellung der Proteinmengen aus 30 mg Schweineleber quantifiziert im Lysat, nach Fällung aus dem Lysat gemäß dem erfindungsgemäßen Verfahren und schließlich im RNA Eluat nach der Reinigung;
- Figur 16: ein Elektropherogramm der Gesamt-RNA Fraktion nach Reinigung aus 30 mg Schweinemilz mittels AllPrep DNA/RNA/Protein (Qiagen, Art. Nr. 80004);
- Figur 17: ein Elektropherogramm der gepoolten Fraktionen langer und kurzer RNA nach Reinigung aus 30 mg Schweinemilz gemäß dem erfindungsgemäßen Verfahren;

### Beispiel 1:

### Isolierung von Protein, langer RNA und kurzer RNA aus Gewebe

### Zelllyse

30 mg Schweineleber wurden mit 300 µl Lysepuffer (5 M Guanidiniumthiocyanat, 1% β-Mercaptoethanol, 30 mM Natriumcitrat pH 5) versetzt und mittels Mikropistill so lange mechanisch zerkleinert bis sich das Gewebe nahezu vollständig aufgelöst hat. Das so entstandene Lysat wurde anschließend durch Filtration von nicht lysierten, festen Bestandteilen getrennt. Dazu wurde das Lysat, wie im Standard Protokoll zu NucleoSpin^{®} RNA II (Kit zur Isolierung von Gesamt-RNA, Macherey-Nagel, Art. Nr, 740955.50) beschrieben, durch einen NucleoSpin^{®} Filter zentrifugiert.

### Entfernung der langen Nukleinsäuren

Das klare Filtrat wurde mit Ethanol (96-100%) auf eine Ethanol-Konzentration von 25% eingestellt und sorgfältig gemischt, um die Bindebedingung für lange Nukleinsäuren einzustellen. Die folgenden Schritte wurden gemäß NucleoSpin^{®} RNA II Protokoll (Rev. 08 / Oktober 2007) durchgeführt. Eine NucleoSpin^{®} RNA II Säule wurde mit der Probe beladen und die langen Nukleinsäuren durch Zentrifugation gebunden. Hierbei handelt es sich um einen Hohlkörper mit Ein- und Auslassöffnung in den eine Silicamembran als fester Träger eingebracht ist. Anschließend folgte der DNase Verdau der auf der Säule gebundenen DNA sowie das Waschen und die Elution der auf der Säule verbliebenen langen RNA gemäß Protokoll.

### Isolierung des Proteins

Der Durchlauf nach der ersten NucleoSpin^{®} RNA II Säule von ca. 400 µl wurde mit einem ¾ Volumen (ca. 300 µl) eines Protein Fällungspuffer (900 mM ZnCl₂, 900 mM Natriumacetat pH 5.0) versetzt und gut gemischt. Das entstandene weiße, flockige Proteinpräzipitat wurde nun durch Zentrifugation (5 min, RT, 14000xg) und Abnehmen des Überstandes isoliert.

### Isolierung der kleinen RNA

Der klare Überstand der Probe nach Entfernung der Proteine wurde mit Tetrahydrofuran (THF) versetzt, so dass der Anteil des THF ca. 55% betrug. Eine NucleoSpin^{®} RNA II Säule in einem Collection Tube (2 ml) wurde dann mit der Probe beladen und zentrifugiert. Dabei binden die kurzen Nukleinsäuren an den Silicaträger (1 min, RT, 11000xg). Die Säule wurde mit 600 µl eines Chaotropsalzwaschpuffers (1,33 M Guanidiniumthiocyanat, 30 mM Natriumcitrat pH 7, 66% EtOH) beladen und zentrifugiert (1 min, 11000xg). Es folgte ein zweiter Waschschritt mit 600 µl eines Chaotropsalz-freien Waschpuffers (2,5 mM Tris/HCl pH 7,5, 20 mM NaCl, 80% Ethanol). Die Säule wurde dann erneut mit 200 µl dieses Puffers beladen und durch Zentrifugation (2 min, RT, 11000xg) getrocknet. Die kurze RNA wurde dann mit 50 µl RNase-freiem Wasser eluiert.

### Charakterisierung der isolierten RNA und des Proteins

Je 1 µl der langen und der kurzen RNA Eluate wurden mit Hilfe eines Agilent 2100 Bioanalyzers auf einem Agilent RNA 6000 Nano Kit Chip (Art. Nr. 5067-1511) einer Elektrophorese unterzogen.

Die isolierten Fraktionen der kleinen und der langen RNA wurden durch Absorptionsmessung bei 260 nm quantifiziert (A₂₆₀ = 1 entspricht einer Konzentration von ca. 40 µg/ml in einer 1 cm Standardküvette). Die Reinheit wurde über die Quotienten A₂₆₀/A₂₈₀ erfasst.

Repräsentativ für alle isolierten miRNAs in der Fraktion kleiner RNA wurde miR-16 mittels quantitativer RT-PCR nach Herstellerangaben (Applied Biosystems Art. Nr. 4373121, 4324018 und 4366596) in einem Roche LightCycler bestimmt. Zur Messung wurde die Probe ca. 1:10⁵ verdünnt.

Die isolierten/gefällten Proteine wurden nach dem Standard Protokoll NucleoSpin^{®} RNA/Protein (Macherey-Nagel, Art. Nr. 740933.50) gewaschen und getrocknet. Die Proteine wurden in dem Protein Loading Buffer (PLB) des Kits gelöst und einer SDS-PAGE unterzogen.

### Ergebnis

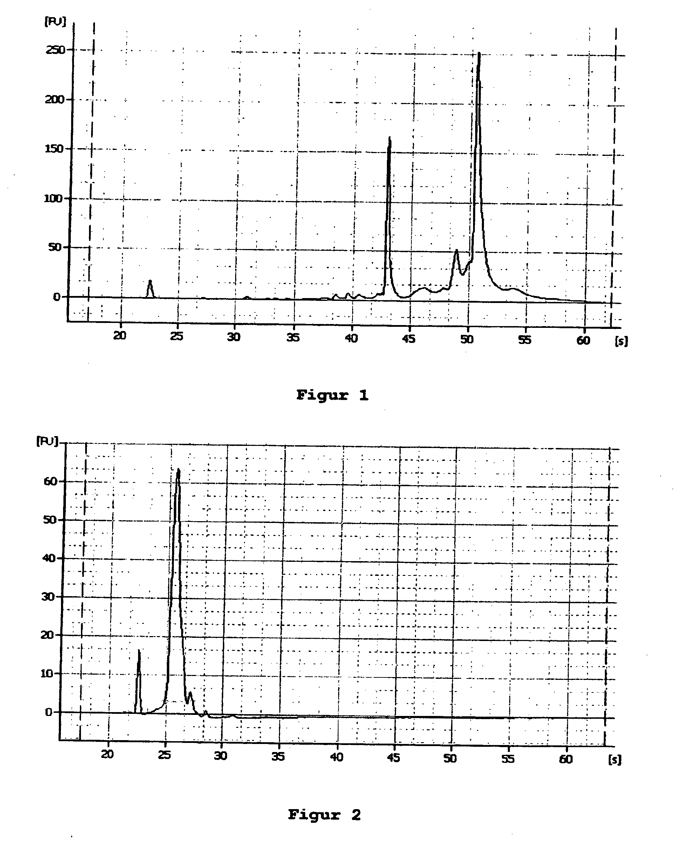

Figur 1 zeigt ein typisches Elektropherogramm tierischer RNA mit deutlichen 18S und 28S rRNA Peaks. Die in größeren Mengen vorkommenden kurzen RNAs sind hier nicht zu sehen. Sie zeigen sich dagegen deutlich im Elektropherogramm der gereinigten Fraktion kurzer RNA in Figur 2 bei etwa 25-30 s kurz hinter dem Markerpeak. Reste langer RNA, z. B. die in Figur 1 dominierenden rRNA Peaks sind nicht zu sehen. Somit zeigen die beiden Abbildungen eine vollständige Fraktionierung in kurze und lange RNA.

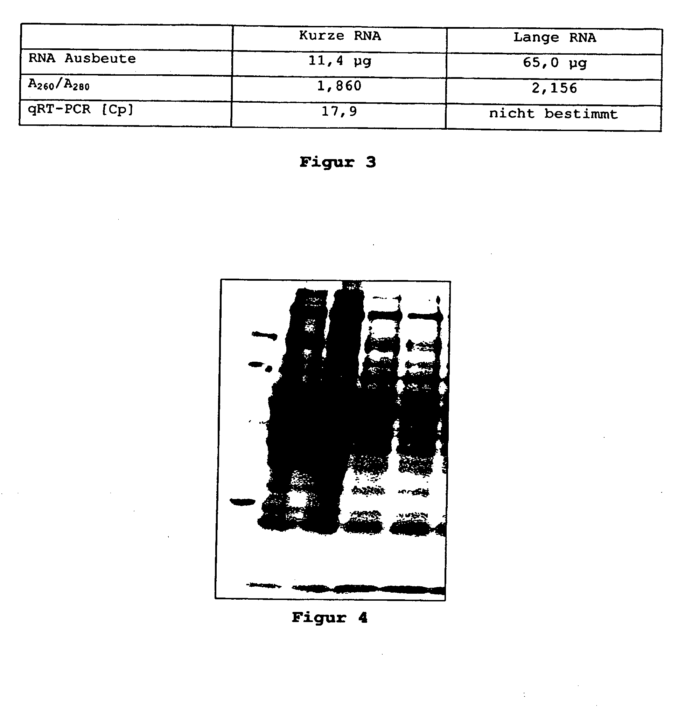

RNA Ausbeuten und Reinheiten der beiden Fraktionen sind in Figur 3 zusammengefasst. Der Cp-Wert von 17,9 in der qRT-PCR für die 1:10⁵ verdünnte Fraktion der kurzen RNA zeigt die hohe Konzentration der isolierten miRNAs. Die Negativkontrolle dieses TaqMan^{®} PCR Systems liefert auch nach 50 Zyklen kein Produkt. Die Abwesenheit von PCR Inhibitoren wurde durch Verdünnungsreihen und Bestimmung der Amplifikationseffizienz belegt (Daten nicht gezeigt).

Figur 4 zeigt im SDS-PAGE, dass durch die Fällung nicht etwa nur kleine oder nur große Proteine gefällt wurden, sondern, dass die Gesamtheit aller zellulären Proteine, unabhängig von ihrer Größe, sichtbar ist. Dies ist eine wichtige Voraussetzung, wenn die Proteine nachfolgend weiter analysiert werden sollen (z.B. Western-Blot).

### Beispiel 2:

### Extraktion und Reinigung von miRNAs aus Zellen mit Silicapartikel

Zur Darstellung der Anwendbarkeit des Verfahrens auf andere Probenmaterialien und Bindematrices wurde kurze und lange RNA sowie Protein aus 3x10⁶ HeLa Zellen mit Hilfe von Silicapartikeln isoliert. Die Lyse und Aufreinigung erfolgte wie im Ausführungsbeispiel 1 beschrieben. An Stelle der NucleoSpin^{®} RNA II Säulen mit Silicamembran wurden jedoch Silicapartikel als Bindematrix verwendet.

Zur Bindung der Nukleinsäuren und für wasch- und Elutionsschritte wurden die Silicapartikel in der jeweiligen Probe bzw. dem jeweiligen Puffer suspendiert. Für die Bindung und Elution wurde 5 min, für die Waschschritte nur 1 min bei Raumtemperatur inkubiert. Nach einer Zentrifugation zur Sedimentierung der Partikel wurde der Überstand abgenommen und ggf. in ein neues Gefäß überführt.

Die Charakterisierung erfolgte wie in Beispiel 1 ausgeführt.

### Ergebnis

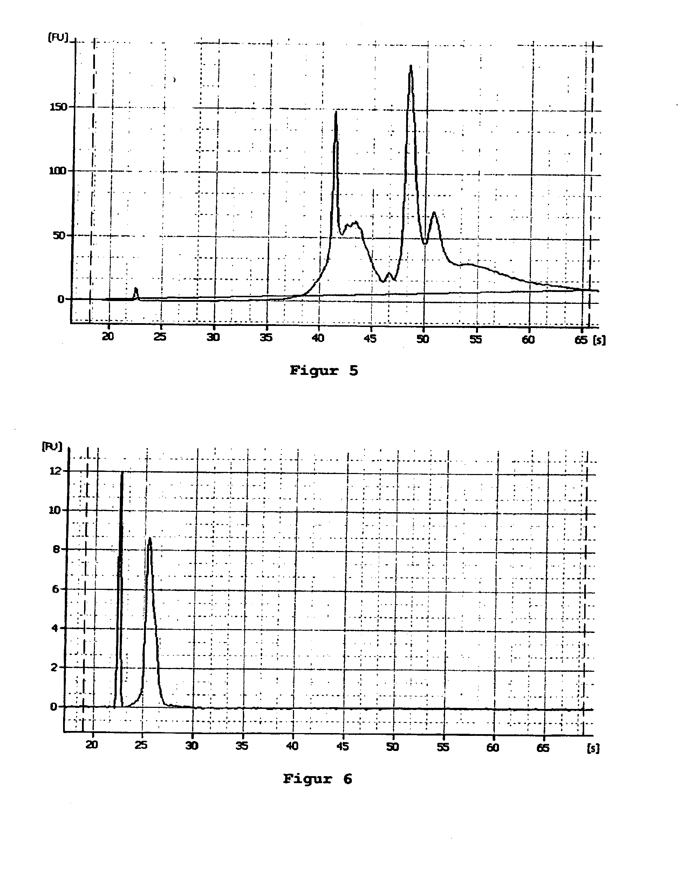

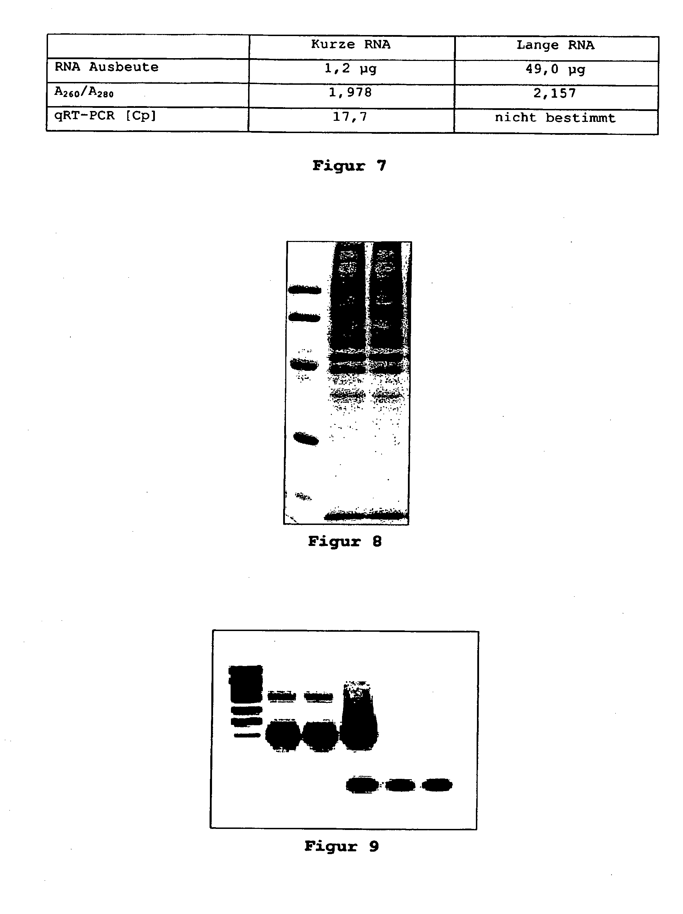

Analog zu Ausführungsbeispiel 1 wurden auch mit Silicapartikel als fester Phase zwei Fraktionen erhalten. Die lange RNA ist in Figur 5, die Kurze in Figur 6 dargestellt. Eine tabellarische Zusammenfassung der Ausbeuten und Reinheiten findet sich in Figur 7. Die mittels SDS-PAGE aufgetrennte Proteinfraktion ist in Figur 8 gezeigt.

### Beispiel 3:

### Reinigung von siRNAs aus einem in vitro Verdau mit DICER

Aus einem in vitro Reaktionsansatz, bei dem lange doppelsträngige RNA (ca. 400 bp) durch das Enzym DICER zu kleiner doppelsträngiger siRNA geschnitten wurde, soll die ungeschnittene lange RNA sowie das DICER Enzym vollständig abgereinigt werden. Durch Variation der Mengen von Ethanol, Lysepuffer und Protein-Fällungspuffer wird der Cut-off nach unten verschoben, so dass die kleine siRNA sauber isoliert werden kann.

### Entfernung der langen dsRNA

150 µl eines in vitro Reaktionsansatzes wurden mit 150 µl Lysepuffer (s. Beispiel 1) und 200 µl Ethanol (96-100%) versetzt. Eine NucleoSpin^{®} RNA II Säule wurde mit der Probe beladen und die lange dsRNA durch Zentrifugation gebunden. Die lange dsRNA wurde nach dem Binden wie die kurze RNA des Ausführungsbeispiels 1 weiter aufgereinigt (waschen und eluieren).

### Reinigung der siRNA

Der Durchlauf wird mit 100 µl Protein Fällungspuffer versetzt, allerdings wurde an dieser Stelle auf die Proteinentfernung verzichtet, da der Reaktionsansatz nur minimale Mengen an Protein enthielt. Die Probe wurde dann mit 800 µl Dioxan versetzt und auf eine zweite NucleoSpin^{®} RNA II Säule geladen. Die gebundene siRNA wurde wie die kurze RNA des Ausführungsbeispiels 1 weiter aufgereinigt.

### Charakterisierung der isolierten. RNA

Die beiden Eluate mit getrennter langer dsRNA und siRNA wurden auf einem 1% TAE EtBr Agarosegel (1 h, 75 V) analysiert.

### Ergebnis

In Figur 9 ist die erfolgreiche Fraktionierung eines in vitro Reaktionsansatzes (Spur 4) zu sehen. Spur 5 und 6 zeigen saubere Fraktionen des 21 bp kurzen, doppelsträngigen siRNA Produktes, das nun völlig frei ist von der 400 bp langen Ausgangs-RNA. Diese ist ohne Reste der kurzen siRNA in Spur 2 und 3 dargestellt.

### Beispiel 4:

### Reinigung von langer RNA vor und nach der Proteinfällung

Dieser Versuch zeigt, dass lange RNA und DNA bei der Proteinfällung vollständig präzipitiert werden. Überraschender weise wurde gefunden, dass kurze RNA dabei in Lösung bleibt und anschließend aufgereinigt werden kann.

### Isolierung langer RNA vor der proteinfällung

Genau wie im Ausführungsbeispiel 1 wurden drei Proben lysiert, mit Ethanol versetzt und auf NucleoSpin^{®} RNA II Säulen geladen. Die DNA auf den Säulen wurde wie beschrieben verdaut, die Säulen gewaschen und die lange RNA eluiert. Auf die Fällung des Proteins und die Aufreinigung der kurzen RNA aus dem Filtrat wurde verzichtet,

### Isolierung langer RNA nach der Proteinfällung

Drei weitere Proben wurden ebenfalls wie im Ausführungsbeispiel 1 beschrieben lysiert und mit Ethanol versetzt. Die Entfernung der langen Nukleinsäuren über die NucleoS-pin^{®} RNA II Säule wurde jedoch übersprungen und es wurde direkt die beschriebene Proteinfällung durchgeführt. Nach Abtrennung des Proteinpräzipitates wurde das klare Lysat auf eine Endkonzentration von 55% THF eingestellt. Diese Konzentration an organischem Lösungsmittel reicht wie im Beispiel 1 gezeigt aus, um sogar kleinste miRNAs zu binden, muss also demnach erst recht auch zur Bindung langer RNA führen. Die Probe wurde auf eine NucleoSpin^{®} RNA II Säule geladen und die Nukleinsäuren wurden durch Zentrifugation (1 min, RT, 11000xg) gebunden. Wie die ersten drei Proben dieses Ausführungsbeispiels wurden die gebundenen Nukleinsäuren einem DNase Verdau unterzogen und anschließend gewaschen und eluiert.

### Ouantifizierung der langen RNA

Alle sechs Eluate wurden wie im Ausführungsbeispiel 1 beschrieben durch Absorptionsmessung bei 260 nm quantifiziert.

### Ergebnis

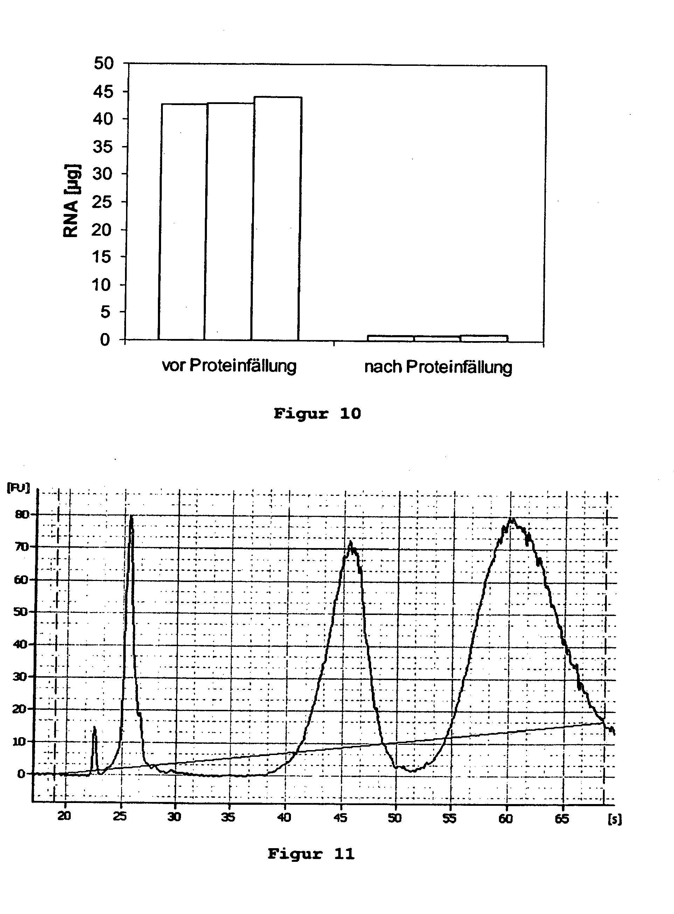

Wie Figur 10 deutlich zeigt, werden die langen RNAs im Zuge der Proteinfällung nahezu quantitativ mit ausgefällt. Die kurzen RNAs hingegen bleiben überraschenderweise in Lösung und lassen sich, wie in Beispiel 1 gezeigt, nachfolgend reinigen.

### Beispiel 5:

### Extraktion und Reinigung von RNAs nach Proteinfällung mit variabler Zn-Konzentration

Ausführungsbeispiel 4 hat gezeigt, dass lange RNA durch Zink zusammen mit dem Protein gefällt wird, kurze RNA jedoch in Lösung bleibt. Dieses Beispiel demonstriert nun genauer den Zusammenhang zwischen Zinkkonzentration und der dadurch gefällten RNA-Längen.

### Reinigung kurzer RNA

Das Ausführungsbeispiel 1 wurde exakt wiederholt, wobei jedoch die Zinkmenge im Protein Fällungspuffer so eingestellt wurde, dass sich nach Zugabe zum Lysat eine Endkonzentration von 200, 400 oder 600 mM ergab.

### Charakterisierung der isolierten RNA

Wie im Ausführungsbeispiel 1 beschrieben wurden die Eluate mit Hilfe eines Agilent 2100 Bioanalyzers analysiert und über UV-VIS und qRT-PCR quantifiziert

### Erqebnis :

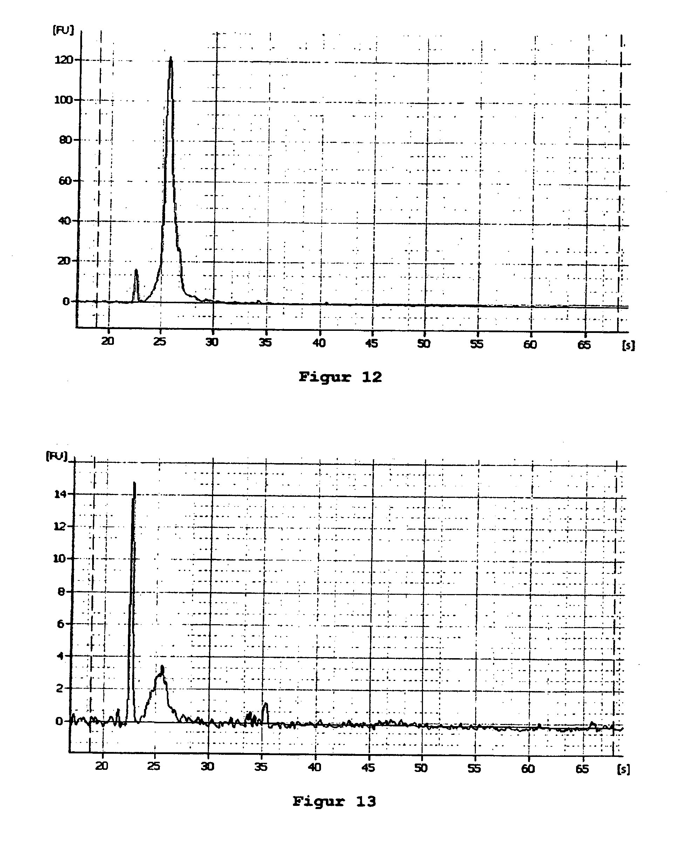

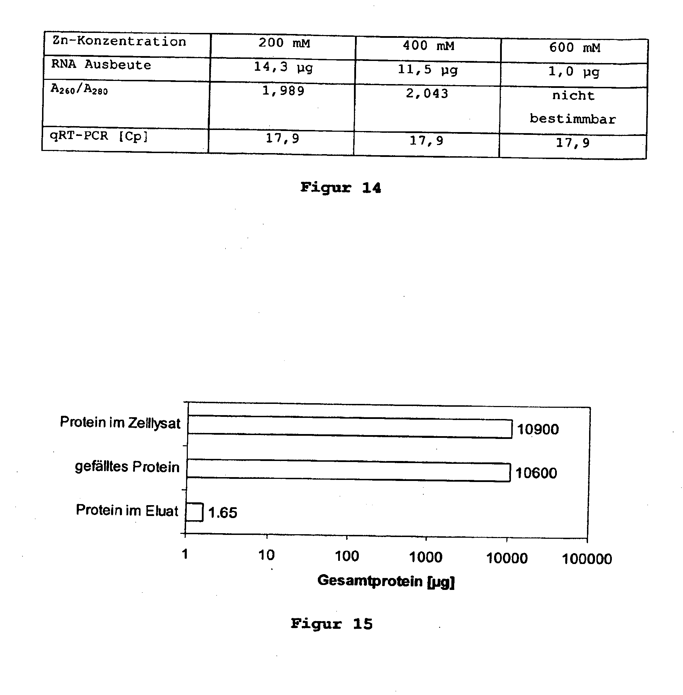

Wie Figuren 11, 12 und 13 zeigen, folgt die Isolierung der kurzen RNAs für die zinkkonzentration einer Optimumskurve. Mit steigendem Zinkgehalt werden im Zuge der Proteinpräzipitation auch zunehmend kürzere Nukleinsäurefraktionen mit ausgefällt. So sind in Figur 11 mit 200 mM Zink neben den kleinen RNAs bei etwa 25 s auch noch breite Peaks für genomische DNA zu sehen. Die genomische DNA fehlt bereits bei 400 mM (Figur 12), ein Hinweis, dass die langen Nukleinsäuren unter diesen Bedingungen mit dem Protein ausfallen. Wird das Zink auf 600 mM erhöht (Figur 13), so nimmt auch die Ausbeute an der Fraktion der kurzen RNAs deutlich ab. Betroffen sind insb. RNA Spezies wie etwa tRNA oder 5.8S rRNA die bei den kurzen Retentionszeiten im Elektropherogramm noch sichtbar sind. Die miRNAs sind hingegen nicht betroffen und werden selbst bei 600 mM Zink nicht ausgefällt, wie die Quantifizierung mittels qRT-PCR in Figur 14 zeigt. Unabhängig von der Zinkkonzentration wurden identische miRNA Konzentrationen (sichtbar am gleichen Cp-Wert) ermittelt.

### Beispiel 6:

### Proteinbilanz

Dieses Ausführungsbeispiel belegt die weitgehend vollständige Entfernung der Proteine durch die Fällung mit Zink.

### Isolierung des Proteins und der kurzen RNA

60 mg Schweineleber wurden in 600 µl Lysepuffer wie im Ausführungsbeispiel 1 beschrieben lysiert und geklärt. Aus 300 µl Lysat wurde wie beschrieben das Protein und die kurze RNA gewonnen.

### Proteinbestimmung

Das gefällte Protein wurde wieder in 300 µl Lysepuffer gelöst und zusammen mit ungefälltem Lysat einer Proteinbestimmung unterzogen. Dazu wurden beide Proben 1:100 in Lysepuffer verdünnt und für den verwendeten Micro BCA Protein Assay Reagent Kit (Pierce, Art. Nr. 23235) eine Eichgerade in Lysepuffer angesetzt.

Im gleichen Ansatz wurde das Restprotein in der Fraktion der kurzen RNA gegen eine Eichgerade in Wasser bestimmt.

### Ergebnis

Für 300 µl Ausgangslysat ergab sich eine Proteinkonzentration von 36,3 µg/µl und damit eine Gesamtproteinmenge von ca. 10.9 mg aus 30 mg Gewebe (Figur 15).

Für das gefällte und wieder gelöste Protein ergab sich eine Konzentration von 35,2 µg/µl in 300 µl und damit eine Gesamtproteinmenge von 10.6 mg.

Der Proteingehalt in der Fraktion kurzer RNA betrug nur noch 0,033 µg/µl n 50 µl Eluat und damit eine Gesamtproteinmenge von 1,65 µg.

Die Konzentration wurde also ca. um den Faktor 1000 und die absolute Menge um ca. den Faktor 6600 verringert, Bezogen auf das Ausgangslysat konnten also durch die Fällung 97 % des Proteins entfernt und einer weiteren Analyse zugänglich gemacht werden.

### Beispiel 7 (Vergleichsbeispiel):

### Abreicherung kurzer RNA durch das Olagen AllPrep DNA/RNA/Protein_Kit

Dieses Ausführungsbeispiel zeigt, dass bei simultaner Reinigung von RNA und Protein aus einer Probe nach dem Stand der Technik die kurzen RNAs mit einem Cut-off von ca. 200 Basen verloren gehen.

### Isolierung der RNA

Aus 30 mg Schweinemilz wurde nach dem Herstellerprotokoll des AllPrep DNA/RNA/Protein Kits (Qiagen, Art. Nr. 80004) RNA gewonnen und in 100 µl Puffer eluiert.

Parallel dazu wurden aus 30 mg Schweinemilz nach dem erfindungsgemäßen Verfahren und wie in Ausführungsbeispiel 1 beschrieben, eine 50 µl Fraktion mit langer RNA und eine 50 µl Fraktion mit kurzer RNA isoliert und anschließend gepoolt.

### Charakterisierung der RNA

Beide Proben wurden wie im Ausführungsbeispiel 1 beschrieben mit Hilfe eines Agilent Bioanalyzers untersucht.

### Ergebnis

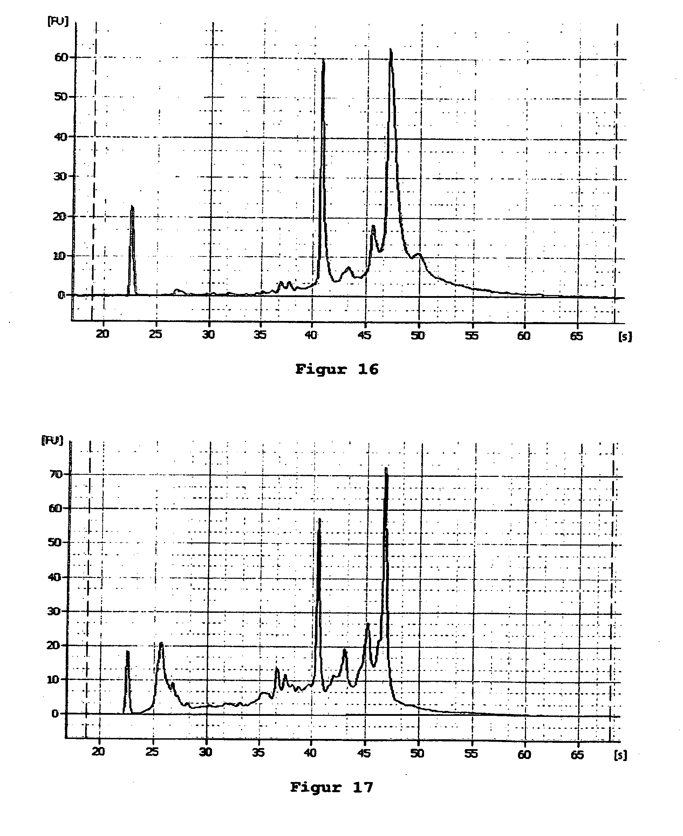

Figur 16 zeigt deutlich, dass mit dem Qiagen AllPrep DNA/RNA/Protein Kit fast die gesamte RNA im Bereich bis 200 Basen verloren geht (fehlende/kleine Peaks bei ca. 25 s), während das erfindungsgemäße Verfahren kurze RNAs nahezu quantitativ aufreinigt (großer Peak der kurzen RNAs bei ca. 25 s., Figur 17).

## Patentansprüche

1. Verfahren zur Gewinnung von zumindest kurzer RNA mit bis zu 200 Nukleotiden mit zumindest folgenden Schritten:
a) Bereitstellen einer biologischen Lösung, enthaltend zumindest kurze RNA sowie Proteine und/oder lange Nukleinsäuren (lange RNA und DNA);
b) Entfernung der Proteine und der langen Nukleinsäuren aus der Lösung, wobei zumindest die Proteine gefällt werden und die Lösung zur Fällung der Proteine mit zweiwertigen Metallionen versetzt wird, wobei die Konzentration der Metallionen auf 0,2 bis 0,6 M eingestellt wird;
c) Adsorbieren der kurzen RNA an einem festen (ersten) Träger nach dem Fällen der Proteine;
d) Gewinnung der kurzen RNA durch Desorption von dem Träger.

2. Verfahren nach Anspruch 1, **dadurch gekennzeichnet, dass** die Proteine und die langen Nukleinsäuren von einander getrennt werden und die Proteine und/oder die lange RNA gewonnen wird bzw. werden.

3. Verfahren nach Anspruch 1 oder 2, **dadurch gekennzeichnet, dass** die langen,Nukleinsäuren vor oder mit der Fällung der Proteine aus der Lösung entfernt werden.

4. Verfahren nach Anspruch 2 oder 3, **dadurch gekennzeichnet, dass** die langen Nukleinsäuren an einen festen (zweiten) Träger adsorbiert werden.

5. Verfahren nach Anspruch 4, **dadurch gekennzeichnet, dass** die Lösung zur Adsorption der langen Nukleinsäuren mit einem organischen, insbesondere wassermischbaren Lösungsmittel in einer solchen Konzentration versetzt wird, dass nur die langen Nukleinsäuren an dem zweiten Träger adsorbieren, wobei die Konzentration des organischen Lösungsmittels insbesondere auf einen Bereich von 15 bis 40 Vol.%, vorzugsweise 20 bis 30 Vol.%, besonders bevorzugt 25 Vol.% jeweils bezogen auf die mit dem bzw. den Lösungsmittel(n) versetzte Lösung, eingestellt wird.

6. Verfahren nach Anspruch 4 oder 5, **dadurch gekennzeichnet, dass** die Lösung zur Adsorption der langen Nukleinsäuren mit einem Salz hoher Ionenstärke, insbesondere einem chaotropen Salz oder mehreren chaotropen Salzen versetzt wird, dessen Konzentration in der Lösung vorzugsweise auf einen Bereich von 1 bis 10 M eingestellt ist.

7. Verfahren nach einem der Ansprüche 4 bis 6, **dadurch gekennzeichnet, dass** von den an den zweiten Träger adsorbierten Nukleinsäuren DNA, insbesondere durch DNase-Verdau entfernt und die verbliebene lange RNA isoliert wird, vor allem, gegebenenfalls nach einem Waschvorgang, von dem zweiten Träger eluiert werden.

8. Verfahren nach einem der Ansprüche 1 bis 7, **dadurch gekennzeichnet, dass** die Lösung zur Fällung der Proteine mit Metallionen versetzt wird, die ausgewählt sind aus der Gruppe der Elemente Fe, Co, Ni, Cu, Zn, Cd, Hg, Pb und Ba, und/ oder die Konzentration der Metallionen insbesondere auf 0,3 bis 0,4 M eingestellt wird.

9. Verfahren nach einem der Ansprüche 1 bis 8, **dadurch gekennzeichnet, dass** zur Adsorption der kurzen RNA eine Lösung verwendet wird, die ein organisches Lösungsmittel in hoher Konzentration enthält, wobei die Konzentration insbesondere auf einen Wert von 30 bis 80 Vol.%, vorzugsweise 40 bis 70 Vol.%, besonders bevorzugt 50 bis 60 Vol.%, jeweils bezogen auf die mit dem Lösungsmittel versetzte Lösung, eingestellt wird und/oder als organisches Lösungsmittel ein nicht-alkoholisches, wassermischbares Lösungsmittel verwendet wird, das z.B. aus der Gruppe ausgewählt wird, zu der Aceton, Acetonitril, Dimethylsulfoxid (DMSO) Tetrahydrofuran (THF), Dioxan und Dimethylformamid (DMF) zählt.

10. Kit für die Durchführung des Verfahrens nach einem der Ansprüche 1 bis 9, enthaltend mindestens
a) zumindest einen festen Träger zur Adsorption von Nukleinsäuren;
b) ein proteinfällendes Reagenz enthaltend zweiwertige Metallionen;
c) zumindest eine Bindesubstanz zur selektiven Adsorption von kurzer RNA an einem festen Träger, wobei die Bindesubstanz ein wassermischbares und nicht-alkoholisches organisches Lösungsmittel ist;
d) eine Anleitung mit den Verfahrensschritten nach einem der Ansprüche 1 bis 9.

11. Kit nach Anspruch 10, **dadurch gekennzeichnet, dass** zumindest eine Bindesubstanz zur Einstellung der Bindebedingungen für die selektive Adsorption von langen Nukleinsäuren an einem festen Träger, insbesondere ein wassermischbares, organisches Lösungsmittel und/oder ein Salz aus einem oder mehreren chaotropen Salzen als zumindest eine Bindesubstanz vorhanden ist und/oder der Kit eine DNase für einen DNA-Verdau aufweist.

12. Kit nach einem der Ansprüche 10 oder 11, **dadurch gekennzeichnet, dass** die zweiwertigen Metallionen aus der Gruppe der Elemente bestehen, zu denen Fe, Co, Ni, Cu, Zn, Cd, Hg, Pb und Ba gehören.

13. Kit nach einem der Ansprüche 10 bis 12, **dadurch gekennzeichnet, dass** das wassermischbare und nichtalkoholische organische Lösungsmittel aus der Gruppe ausgewählt ist, zu der Aceton, Acetonitril, Dimethylsulfoxid (DMSO), Tetrahydrofuran (THF), Dioxan und Dimethylformamid (DMF) gehören.

14. Kit nach einem der Ansprüche 10 bis 13, **dadurch gekennzeichnet, dass** als feste Träger Partikel, auch in Form von Pulver oder Suspensionen, Polymeren, Membranen, Filterschichten, Fritten, Vliese oder Träger in Form eines Monolithen vorhanden sind und/oder das Material für den festen Träger Silica, Glas, Quarz, Zeolite oder Mischungen daraus und/oder magnetische Partikel, vorzugsweise mit Silica, Glas, Quarz oder Zeoliten beschichtet, ist.

15. Kit nach einem der Ansprüche 10 bis 14, **dadurch gekennzeichnet, dass** zumindest ein Elutionsreagenz zur Desorption zumindest der kurzen RNA von dem Träger und/oder zumindest ein Waschpuffer vorhanden ist.

## Claims

1. A method for recovering at least short with up to 200 nucleotides RNA, having at least the following steps:
a) making available a biological solution containing at least short RNA as well as proteins and/or long nucleic acids (long RNA and DNA);
b) removing the proteins and the long nucleic acids from the solution, at least the proteins being precipitated and the solution for precipitating of the proteins has metal ions added to it, wherein the concentration of the metal ions is adjusted to 0.2 to 0.6 M;
c) adsorbing the short RNA onto a solid (first) carrier after precipitation of the proteins;
d) recovering the short RNA by desorption from the carrier.

2. The method according to claim 1, wherein the proteins and the long nucleic acids are separated from one another, and the proteins and/or the long RNA is/are recovered.

3. The method according to claim 1 or 2, wherein the long nucleic acids are removed from the solution before or concurrently with precipitation of the proteins.

4. The method according to claim 2 or 3, wherein the long nucleic acids are adsorbed onto a solid (second) carrier.

5. The method according to claim 4, wherein the solution for adsorption of the long nucleic acids has an organic solvent, in particular water-miscible solvent added to it, in a concentration such that only the long nucleic acids adsorb on the second carrier, the concentration of the organic solvent being adjusted in particular to a range from 15 to 40 vol%, by preference 20 to 30 vol%, particularly preferably 25 vol%, based in each case on the solution with the solvent(s) added to it.

6. The method according to claim 4 or 5, wherein the solution for adsorption of the long nucleic acids has added to it a salt of high ionic strength, in particular a chaotropic salt or multiple chaotropic salts, the concentration of which in the solution is adjusted in particular to a range from 1 to 10 M.

7. The method according to any of claims 4 to 6, wherein DNA is removed, in particular by DNase digestion, from the nucleic acids adsorbed onto the second carrier, and the remaining long RNA is isolated, especially is eluted from the second carrier, optionally after a washing operation.

8. The method according to any of claims 1 to 7, wherein the solution for precipitation of the proteins has metal ions added to it, that are chosen from the group of the elements Fe, Co, Ni, Cu, Zn, Cd, Hg, Pb, and Ba and/or the concentration of the metal ions being adjusted to 0.3 to 0.4 M.

9. The method according to any of claims 1 to 8, wherein for adsorption of the short RNA, a solution is used that contains an organic solvent at high concentration, the concentration being adjusted in particular to a value from 30 to 80 vol%, by preference 40 to 70 vol%, particularly preferably 50 to 60 vol%, based in each case on the solution with the solvent added to it; the organic solvent preferable being a nonalcoholic, water-miscible solvent, which is selected, for example, from the group that includes acetone, acetonitrile, dimethyl sulfoxide (DMSO), tetrahydrofuran (THF), dioxan, and dimethylformamide, is used as an organic solvent.

10. A kit for carrying out the method according to any of claims 1 to 9, at least comprising
a) at least one solid carrier for the adsorption of nucleic acids;
b) a protein-precipitating reagent;
c) at least one binding substance for selective adsorption of short RNA on a solid carrier;
d) instructions having the method steps according to one of Claims 1 to 9.

11. The kit according to claim 10, wherein at least one binding substance for adjusting the binding conditions for selective adsorption of long nucleic acids on a solid carrier, in particular a water-miscible organic solvent and/or a salt from among one or more chaotropic salts, is present as at least one binding substance, and/or the kit comprises a DNase for DNA digestion.

12. The kit according to claim 10 or 11, wherein a protein-precipitating reagent containing metal ions is present in the kit, the metal ions being made up of the group of elements to which Fe, Co, Ni, Cu, Zn, Cd, Hg, Pb, and Ba belong.

13. The kit according to any of claims 10 to 12, wherein the organic solvent that is water-miscible and nonalcoholic, is selected e.g. from the group to which acetone, acetonitrile, dimethyl sulfoxide (DMSO), tetrahydrofuran (THF), dioxan, and dimethylformamide (DMF) belong.

14. The kit according to any of the claims 10 to 13, wherein particles, including in the form of powders or suspensions, polymers, membranes, filter layers, frits, nonwoven fabrics, or carriers in the form of a monolith, are present as solid carriers, and/or the material for the solid carrier is silica, glass, quartz, zeolites, or mixtures thereof, and/or magnetic particles preferably coated with silica, glass, quartz, or zeolites.

15. The kit according to any of the claims 10 to 14, wherein at least one elution reagent, for desorption of at least the short RNA from the carrier, and/or at least one washing buffer is present.

## Revendications

1. Procédé d'obtention d'un ARN au moins petit avec jusqu'à 200 nucléotides comprenant au moins les étapes suivantes :
a. Préparation d'une solution biologique contenant au moins des petits ARN ainsi que des protéines ou de longs acides nucléiques (ARN et ADN longs) ;
b. Extraction des protéines et des acides nucléiques longs de la solution, au moment de quoi au moins les protéines sont précipitées et la solution mélangées, aux fins de la précipitation des protéines, avec des ions métalliques bivalents, la concentration des ions métalliques étant réglée à un niveau compris entre 0,2 et 0,6 M ;
c. Adsorption des ARN courts avec un (premier) support fixe après la précipitation des protéines ;
d. Obtention des petits ARN par la désorption du support.

2. Procédé selon la revendication 1, **caractérisé en ce que** les protéines et les acides nucléiques longs sont séparées et que les protéines ou l'ARN long sont/est obtenu.

3. Procédé selon la revendication 1 ou 2, **caractérisé en ce que** les acides nucléiques longs sont extraits de la solution avant ou pendant la précipitation des protéines.

4. Procédé selon la revendication 2 ou 3, **caractérisé en ce que** les acides nucléiques longs sont adsorbés à un (deuxième) support fixe.

5. Procédé selon la revendication 4, **caractérisé en ce que**, aux fins de l'adsorption des acides nucléiques longs, la solution est mélangée à un solvant organique, plus précisément hydrosoluble, dans une telle concentration que seuls les acides nucléiques longs sont adsorbés au deuxième support fixe, la concentration du solvant organique étant comprise plus précisément entre 15 et 40 de % volumique, de préférence entre 20 et 30 % volumique et idéalement à 25 % volumique par rapport à la solution mélangée avec le ou les solvant(s).

6. Procédé selon la revendication 4 ou 5, **caractérisé en ce que** la solution, aux fins de l'adsorption des acides nucléiques longs, est mélangée à un sel de force ionique élevée, plus précisément un sel chaotropique ou plusieurs seuls chaotropiques dont la centration dans la solution est réglée à un niveau compris entre 1 et 10 M.

7. Procédé selon l'une des revendications 4 à 6, **caractérisé en ce que** de l'ADN est extrait des acides nucléiques adsorbés au deuxième support, plus précisément par digestion à la DNase, et que les ARN longs restants sont isolés et surtout élués du deuxième support, le cas échéant après un nettoyage.

8. Procédé selon l'une des revendications 1 à 7, **caractérisé en ce que**, aux fins de la précipitation des protéines, la solution est mélangée à des ions métalliques sélectionnés parmi le groupe d'éléments Fe, Co, Ni, Cu, Zn, Cd, Hg, Pb et Ba ; ou que la concentration des ions métalliques est réglée plus précisément à un niveau entre 0,3 et 0, 4 M.

9. Procédé selon l'une des revendications 1 à 8, **caractérisé en ce que**, aux fins de l'adsorption des petits ARN, la solution est mélangée à un solvant organique hautement concentré, la concentration étant réglée précisément entre 30 et 80 de % volumique, de préférence entre 40 et 70 % volumique et idéalement entre 50 et 60 % volumique par rapport à la solution mélangée avec le solvant ; ou que le solvant organique utilisé est un solvant organique non alcoolique hydrosoluble qui est, par exemple, sélectionné parmi le groupe comprenant l'acétone, l'acétonitrile, le diméthylsulfoxyde (DMSO), le tétrahydrofurane (THF), le dioxane et le dimethylformamide (DMF).

10. Trousse servant à l'exécution du procédé selon l'une des revendications 1 à 9 comprenant au moins
a. au moins un support fixe pour l'adsorption d'acides nucléiques ;
b. un réactif pour la précipitation des protéines contenant des ions métalliques bivalents ;
c. au moins une substance agglutinante pour l'adsorption sélective d'un ARN court à un support fixe, la substance agglutinante étant un solvant hydrosoluble et non alcoolique ;
d. des instructions comprenant les étapes de procédé selon l'une des revendications 1 à 9.

11. Trousse selon la revendication 10, **caractérisée en ce qu'**au moins une substance agglutinante, plus précisément un solvant hydrosoluble et organique ou un sel provenant d'un ou plusieurs sels chaotripiques, est contenue pour le réglage des conditions de liaison en vue de l'adsorption sélective d'acides nucléiques longs à un support fixe ; ou que la trousse contient une DNasepour la digestion d'ADN.

12. Trousse selon l'une des revendications 10 à 11, **caractérisée en ce que** les ions métalliques bivalents sont compris dans le groupe d'éléments parmi lequel comptent le Fe, Co, Ni, Cu, Zn, Cd, Hg, Pb et Ba.

13. Trousse selon l'une des revendications 10 à 12, **caractérisée en ce que** le solvant organique, hydrosoluble et non alcoolique est sélectionné dans le groupe comprenant l'acétone, l'acétonitrile, le diméthylsulfoxyde (DMSO), le tétrahydrofurane (THF), le dioxane et le dimethylformamide (DMF).

14. Trousse selon l'une des revendications 10 à 13, **caractérisée en ce que**, en tant que supports fixes, sont contenus des particules, également sous la forme de poudre ou de suspensions, des polymères, membranes, couches filtrantes, frites, non-tissés ou des supports sous la forme de monolithes ; ou que le matériau composant le support fixe est recouvert de silice, verre, quartz, zéolithe ou de mélanges de ces derniers ou de particules magnétiques, préférablement de silice, verre, quartz ou de zéolithes.

15. Trousse selon l'une des revendications 10 à 14, **caractérisé en ce qu'**au moins un réactif d'élution servant à la désorption au moins des ARN courts du support ou au moins un tampon de lavage est contenu.
